# EUROPEAN PATENT SPECIFICATION

(11) **EP 1 006 967 B1**
(45) Date of publication and mention of the grant of the patent: **26.10.2005**
(21) Application number: 98903709.8
(22) Date of filing: 28.01.1998
(51) Int. Cl.: A61F 9/007, A61F 9/013

(54) **SHORT PULSE MID-INFRARED PARAMETRIC GENERATOR FOR SURGERY**
KURZGEPULSTER PARAMETRISCHER MITTEL-INFRAROT-GENERATOR ZUR CHIRURGIE
GENERATEUR PARAMETRIQUE CHIRURGICAL D'IMPULSIONS COURTES DANS L'INFRAROUGE MOYEN

(30) Priority: 14.03.1997 US 816097
(43) Date of publication of application: 14.06.2000
(73) Proprietor: VISX INCORPORATED, Santa Clara, CA 95051 (US)
(72) Inventor: TELFAIR, William, B., San Jose, CA 95124 (US); ZENZIE, Henry, Chelmsford, MA 01824 (US); MOULTON, Peter, Concord, MA 01742 (US); HOFFMAN, Hannah, J., Palo Alto, CA 94303 (US)
(74) Representative: Dendorfer, Claus
(86) International application number: PCT/US1998/001406
(87) International publication number: WO 1998/041177

(56) References cited:
- EP-A- 0 770 370
- WO-A-93/14817
- US-A- 5 144 630
- US-A- 5 181 211

## Description

### BACKGROUND OF THE INVENTION

In recent years, photorefractive keratectomy (PRK) techniques for reshaping the cornea of the eye have become widely utilized as an effective means for correcting visual deficiencies. These methods are generally based on volumetric removal of tissue using ultraviolet (UV) radiation, typically from a 193 nm ArF excimer laser. At this short wavelength, the high photon energy causes direct breaking of intramolecular bonds, in a process known as photochemical decomposition. Tissue ablation based on this photochemical mechanism has the advantage of producing minimal collateral thermal damage in cells adjacent to the surgical site. Also, the depth of decomposition is very small, typically less than 1 micron, resulting in accurate tissue removal with minimal risk of damage to underlying structures from UV radiation.

While excimer-based methods have been established as a safe and effective method of corneal ablation, they suffer from a number of deficiencies, including high initial cost and ongoing maintenance costs, large and complex optical beam delivery systems, safety hazards due to the fluorine and ozone gas formation and persistent reliability problems. Furthermore, the potential phototoxicity of high-power UV radiation is still an undetermined risk in excimer-laser-based PRK. In particular, there is concern that the UV radiation poses certain mutagenic and cataractogenic risks due to secondary fluorescence effects.

A recently suggested alternative to the excimer laser for performing corneal refractive surgery involves ablation at mid-infrared wavelengths using, in particular, radiation around 3 µm corresponding to the absorption peak of water, the main constituent of the cornea. The premise underlying interest in such an alternative system is that infrared radiation can be produced with solid-state technology, which would provide easier handling, is cheaper, more compact and has better reliability features while eliminating the potential of any safety concerns due to toxic gases or mutagenic side effects associated with UV wavelengths. One solid state laser in particular, the erbium:YAG (Er:YAG) laser, emits radiation at a wavelength of 2.94 µm, corresponding to an absorption coefficient of over 13000 cm -1 in water. This high absorption results in a relatively small region of impact with potentially less than 2 microns penetration depth. Contrary to the photoablation mechanism associated with the excimer laser, i.e., photochemical decomposition, ablation at the erbium wavelength is attributed to photovaporization, or photothermal evaporation, of water molecules. This process is inherently more efficient than photodecomposition, allowing for removal of up to 3 microns of tissue at a time, resulting in faster surgical operation. Such a system has been suggested for example by T. Seiler and J. Wollensak, "Fundamental Mode Photoablation of the Cornea for Myopic Correction", Lasers and Light in Ophthalmology, 5,4,199-203 (1993). Another system has been described by Cozean et al. in PCT Application No. 93/14817, which relies on a sculpting filter to control the amount of tissue removal using a pulsed 3 µm Er:YAG laser. However, while ophthalmic surgical techniques based on free running or long-pulse erbium lasers have shown some promise, they also suffer from a number of drawbacks principally relating to the fact that the IR radiation causes collateral thermal damage to tissue adjacent to the ablated region, where the size of the damage zone may exceed several microns, resulting in potentially undesirable long term effects.

Recently, it has been recognized that lasers having a pulse duration shorter than a few tens of nanoseconds will demonstrate less dominant thermal effects. In particular, a direct tissue interaction effect known as photospallation has been observed at infrared wavelengths whereby, with shorter pulses, radiation interacts exclusively with the irradiated tissue producing negligible effect upon the adjacent, unirradiated tissue. Photospallation is a photomechanical ablation mechanism which results from the rapid absorption of incident radiation and subsequent expansion by the corneal tissue. This expansion is followed by a bi-polar shock wave that causes removal of tissue. For a detailed description of a method and apparatus for performing corneal surgery that directly exploits the photospallation mechanism to remove tissue, see U.S. Patent Application Serial No. 08/549,385, corresponding to EP-A-0 770 370 which is prior art under Art. 54(3) EPC. The method and apparatus disclosed therein utilize a short-pulse (preferably less than 50 ns) solid state laser emitting mid-infrared radiation, preferably at or around 2.94 µm, scanned over a region of the cornea to allow uniform irradiation of the treatment region using a relatively low-energy laser. As pointed out in the parent application, a desired laser source for this application would have output energy of up to 30 mJ and repetition rates of up to 100 Hz, depending on the details of the delivery system.

An erbium-doped laser operating at 2.94 µm is one option for such a laser source. A compact, reliable Q-switched erbium laser is described in our co-pending patent application Serial No. 08/549,385. While highly attractive because of its simplicity, even with the aid of future diode pumping, it may be difficult to extend the erbium laser operation to high repetition frequencies (in excess of 30 Hz) due to strong thermal birefringence effects. Limitations of the fundamental level dynamics and long upper-laser-level lifetimes may also conspire with peak-power damage to optical component coatings to impose a practical lower limit on the pulse duration of 20 ns or so in an erbium-based laser operating in a Q-switched mode.

Recognizing that it is possible that a shorter pulse (less than 20 ns) may increase the percentage of true photospallative ablation process, and thus further reducing residual contributions to tissue ablation from undesirable thermal effects, it is desirable to construct the shortest pulse solid state mid-infrared laser source that can safely and efficaciously meet the requirements of PRK. Ideally, such a source would also be scalable to high repetition frequencies (approaching 100 Hz) without substantially increasing the expense and complexity of the device or compromising its reliability.

An Optical Parametric Oscillator (OPO) that can downshift the frequency of radiation from a standard neodymium-doped laser, such as Nd:YAG, operating at or about 1.06 µm has been suggested as an alternative approach in our co-pending U.S. patent application Serial No. 08/549,385, to obtaining the desired parameters at mid-IR wavelengths. However, no such device has been available to date that can meet all the requirements of the ophthalmic surgical procedures contemplated. For example, efficient OPOs which are pumped by a 1 micron laser with output in the IR range have been demonstrated in recent years using a number of different nonlinear crystals such as Lithium Niobate (LiNbO₃) and Potassium Titanyl Phosphate (KTiOPO₄ or "KTP"). Examples of parametric oscillation near the 3 µm wavelength of interest include the generation of high-power radiation (8W) at 3.5 µm using LiNbO₃ pumped by a 100 Hz, single-mode pump beam (see A. Englander and R. Lavi, OSA Proceedings on Advanced Solid-State Lasers, Memphis, Tennessee, 1995, p. 163) and demonstration of a 0.2W output at 3.2 µm using KTP in a non-critical phase match configuration (see, for example, K. Kato in IEEE J. Quantum Electronics. 27, 1137 (1991)). Realization of an optical parametric device with output at the desired 2.9 to 3.0 µm wavelength range was considered difficult because the two readily available candidate crystals of LiNbO₃ and KTP exhibit absorption in that wavelength range. Use of LiNbO₃ in particular is not considered feasible because of absorption at or near 3.0 µm due to the OH-band present in the crystal using current growth methods. Other drawbacks of the OPO design include a perceived requirement for powerful and high-beam-quality pump sources that can overcome the high threshold for the onset of a parametric process. Since the effectiveness of increasing the pump power density by focusing the pump beam is limited by the walk-off angle of the nonlinear crystal, the threshold condition cannot be overcome simply by using small pump beam diameters in most crystals. A way to circumvent this problem is to use a crystal that can be non-critically phase-matched (such as KTP), resulting in higher acceptance angles, but this configuration is not possible for a 1 µm pump beam wavelength and with the output wavelength desired for a successful PRK procedure. Non-critical phase-matching with output in the 2.9-3.0 µm range is, however, feasible in KTP (x-cut) pumped at 0.88 to 0.9 µm. Lasers emitting at this wavelength range are, however, more complex and expensive than standard neodymium doped laser at or near 1 micron.

For a medical laser instrument, it is generally not desirable to impose overly stringent requirements on the pump laser, as that would result in more complex and costly systems. Ideally, a multimode gaussian or a top-hat beam profile that is commercially available would be desired. However, prior to the present invention, it was not clear that such a pump beam, which can possess substantial divergence, would produce the requisite output energies without damaging the OPO crystal and/or the coupling optics. Also, in the case of a gaussian spatial profile beam, uneven distribution of the peak power density across the crystal can result in only part of the beam contributing significantly to the parametric generation thereby compromising the efficiency of conversion. Furthermore, absorption in KTP, which is known to be substantial at 3.0 µm, was another issue of concern especially for operation at elevated average power levels and/or high repetition rates. These as well as other reasons prevented the realization to date of an OPO source of pulsed 2.9-3.0 µm radiation of practical output energies and repetition rates.

The present invention discloses a specific apparatus for producing short-pulse radiation at or near 2.94 µm which overcomes the aforementioned difficulties. The apparatus is uniquely suited to performing PRK and other microsurgery procedures at minimal complexity and low cost, thus greatly increasing the availability of such procedures to a large number of people. Furthermore, with certain adjustments to the apparatus, it may be used for certain other ophthalmic procedures where a concentrated pulsed beam at a selected mid-IR wavelength has demonstrated benefits. These procedures include laser sclerostomy, trabeculectomy and surgery of the vitreous and /or the retina. In these procedures means for affecting precise, highly localized tissue ablation are desired. For example, in the case of laser-assisted vitroretinal surgery, the application of mid-IR radiation at 2.94 µm offers the potential of tractionless maneuvers, shallow penetration depths and extreme precision both in transecting vitreous membranes and in ablating requisite epiretinal tissue. See, for example, J.F. Berger, et al. in SPIE, vol. 2673, 1994, p. 146. Furthermore, by utilizing short pulses as disclosed in the present invention, the procedure may be efficaciously conducted at lower fluence levels thus easing requirements on probe geometry. In glaucoma filtration procedures such as ab externo sclerostomy, where a fistula is created from the anterior chamber of the eye into the subconjuctival space, the application of a nanosecond, low energy pulses from an excimer laser at 308 mm proved highly advantageous in treating a number of severely affected patients. See, for example, J. Kampmeier et al. in Ophthalmolge, 90, p. 35-39, 1993. Similar effectiveness of the procedure is expected for mid-IR wavelength due to the high absorption properties of the sclera. The main issue which prevented wider use to date of mid-IR laser radiation in micro-ocular surgery was the lack of a suitable fiber for delivering the energy to the target tissue. However, recent developments in this area culminated in a number of potential fiber technologies including zirconium fluoride, sapphire silver halide and hollow waveguide technologies. With further improvements in damage thresholds, it appears that sufficiently flexible, low loss fibers and appropriate probes may become available in the very near-term that can handle delivery of even short pulse, 3-micron radiation, for lower energy (<10 mJ) applications. The emergence of such fiber delivery systems may also make short pulse, mid-IR radiation highly attractive in general endoscopic microsurgery. In particular, medical procedures such as brain, orthoscopic and spinal cord surgery may benefit from the highly localized effects generated by the photo-mechanical ablation associated with the present system because the delicate nature of the tissues involved places a premium on limiting collateral thermal injury in surrounding tissue. Of course, optimal parameters of the laser may very with the application, tissue type and desired effect. But in this respect, the OPO laser has an advantage in that it offers great flexibility in terms of available outputs including variations in wavelength and pulse duration.

US Patent 5,144,630 discloses several embodiments of a laser apparatus with a basic pulsed laser and at least one nonlinear crystal for frequency conversion. For example, a Nd:YAG laser in combination with a nonlinear KTP or KNbO₃ crystal is disclosed for obtaining a tunable IR wavelength ranging from 1.5 - 4.5 nm. Lasers with such wavelengths are considered to be suitable for a number of medical applications, including ophthalmic surgery and corneal reshaping.

### SUMMARY OF THE INVENTION

It is therefore an object of this invention to provide a new and improved surgical apparatus, that is particularly adapted for performing corneal refractive surgery. It is another object to facilitate a new and improved method of photorefractive laser surgery based on utilizing short-pulse, mid-infrared radiation produced by parametric downconversion of radiation from a neodymium-doped laser, such as Nd:YAG.

The short pulses are viewed as critical to reducing unwanted changes in adjacent tissue and especially thermal effects which can result in undesirable irregular edges of the interaction site produced by the infrared radiation. With sufficiently short pulses, the thermal damage can be reduced to potentially sub-micron levels, resulting in the same clinical indications as ablative photodecomposition produced by deep-UV lasers, commonly used in refractive surgical procedures. Consequently, it is a further aspect of the present invention to provide a laser source with pulse durations shorter than 25 ns at or near 3.0 µm but preferably close to the 2.94 µm water absorption maximum.

It is a further object of this invention to provide a new and improved laser surgical apparatus utilizing an OPO based on a nonlinear crystal such as KTP or its isomorphs for shifting the wavelength of a neodymium-doped laser to the desired mid-infrared wavelength range near 3.0 µm. In an alternative embodiment, a related objective would be to provide a non-critically phased-matched crystal to shift the wavelength from a near-infrared laser source emitting at or around 880-900 nm to the desired 3.0 µm wavelength range.

In yet another object, the OPO cavity parameters are such as to accommodate a readily available pump beam of moderate power while still producing a stable output with pulse energies scalable to the tens of millijoules level. In a preferred embodiment of the OPO laser, pump beams that are single or multi-mode with either gaussian or top-hat spatial profiles and with divergence ranging to many times the diffraction limit would all be accommodated, while maintaining a simple optical configuration with a minimum number of elements.

It is a further object to provide, within the OPO configuration, means for elevating damage thresholds, such that short pulse pump beams with energy outputs over 200 mJ at wavelengths at or near 1-micron can all be accommodated without damage at repetition rates exceeding 10 Hz and preferably approaching 50 Hz. A related object is to provide optimal OPO configurations such that the lowest pump thresholds result for a desired output in the mid-IR range.

It is still another object to provide a new apparatus and method for performing refractive surgery using a fiber or a fiber bundle or some other waveguide means to separate the pump laser from the OPO cavity. The OPO portion could then be mounted to the surgical microscope providing the surgeon with maximal flexibility for delivering the light to the patient's eye.

The invention is defined by the independent claim. The dependent claims define preferred embodiments of the invention.

A more complete understanding of the present invention, as well as further features and advantages of the invention, will be obtained by reference to the detailed description and drawings.

### BRIEF DESCRIPTION OF THE DRAWINGS

Figure 1 is a schematic diagram illustrating a preferred embodiment of the OPO laser device according to the present invention.
Figure 2 is a schematic diagram illustrating an alternative embodiment of the OPO laser source, using an L-shaped configuration.
Figure 3 is a schematic diagram illustrating another alternative embodiment of the OPO using a single-pass pump beam.
Figure 4 is a schematic diagram illustrating yet another alternative embodiment using single-pass pump beams in a ring configuration.
Figure 5 is a schematic diagram illustrating of a preferred embodiment of the OPO laser source where the pump beam is fiber coupled to the OPO.

### DETAILED DESCRIPTION

A mid-IR laser source is disclosed with parameters selected to yield a beam with properties matched to optimal tissue removal based on a photospallation mechanism. Optimally, the laser beam comprises a series of discrete pulses of less than 25 ns in duration, each with energy of greater than 1 mJ emitted at repetition rates of at least 10 Hz, but scalable to over 50 Hz. High repetition rate is required to minimize the duration of the medical procedure while allowing small spot sizes with better overlap parameters to be utilized for improved surgical outcomes. The critical nature of the pulse duration is related to the threshold for the photospallation process, which is expected to be lower as the pulse duration decreases thus allowing for lower energy densities (or, fluences) to be utilized to affect ablation. Generally, the lower the energy density, the less likely it is that thermal damage to tissue surrounding the ablation site will occur. This, in turn, is an important factor in producing highly localized ablation with clinical results similar to what is obtained currently with UV radiation.

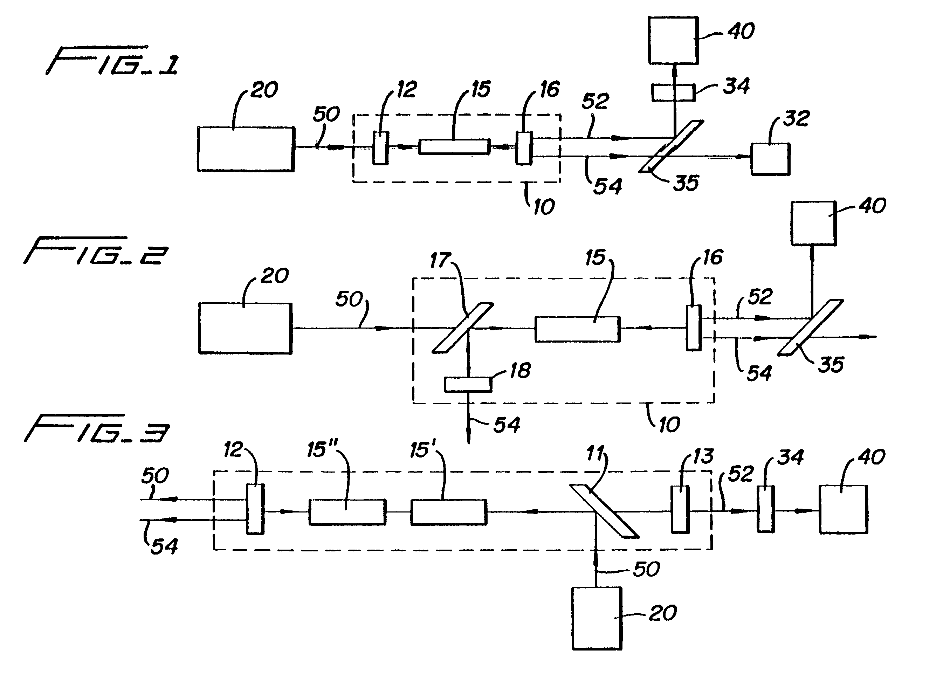

As shown in Fig. 1, a mid-infrared laser source 1 preferably includes a neodymium-doped laser source pump 20, generating a pump beam 50 comprised of short laser pulses (preferably less than 30 ns) at or around 1 micron, which radiation is downconverted to the mid-IR wavelength range through an Optical Parametric Oscillator (OPO) 10. The OPO 10 is shown to include mirrors 12, 16 and a nonlinear crystal 15. The effect of the nonlinear crystal 15 on the laser pulses results in two beams, in a known manner. Specifically, the output of the OPO comprises an idler beam 52 and a signal beam 54. For a detailed description of the operation of one particular OPO, see U.S. Patent No. 5,181,211.

For refractive surgery, the desired wavelengths are those of the idler beam 52, which in the preferred embodiment fall in the range between 2.89 and 2.98 µm. In the example of a Nd:YAG pump beam at 1.064 µm, the corresponding wavelength of the signal beam 54 is between 1.68 and 1.66 µm. It is to be understood, however, that while a wavelength near the 2.94 µm water absorption peak is preferred, especially for PRK applications, idler wavelengths anywhere in the range of approximately 2.75 to just over 3.0 µm fall within the scope of the invention, with the specific wavelength chosen to match the needs of the surgical application.

The idler beam 52 is reflected from dichroic beam splitter 35 and is subsequently directed to beam transfer optics 40, which, in a preferred embodiment may include imaging and scanner means to allow selective removal of tissue at various points on the cornea, thereby causing the cornea to change in a predictable and controlled manner. Such means were disclosed in our co-pending parent application, U.S. Serial No. 08/549,385, and are not considered critical to the present invention. The signal beam 54 is transmitted through the beam splitter 35 to a beam dump 32. Further attenuation of the residual signal beam 54 may be provided by additional reflectors collectively represented as attenuator 34 which may be placed in the path of the idler beam 52 to prevent any coupling of the signal wavelengths from the signal beam 54 into the delivery system 40.

In the embodiment of Fig. 1, the coatings and positioning of the mirrors 12, the crystal 15 and the mirror 16 in the OPO cavity 10 are chosen to comprise a singly resonant oscillator (SRO) configuration optimized for producing the idler wavelengths and with the added feature of using backreflection of the unconverted portion of the pump beam 50 into the crystal for further processing. Thus, mirror 12 is coated for high transmission of wavelengths between 1.0 and 1.1 µm and high reflection of the idler wavelengths between 2.8 and 3.0 µm. Mirror 16 is coated to have partial reflectance for wavelengths between 2.8 and 3.0 µm and high transmission at the 1.65 to 1.7 µm wavelengths characteristic of the signal beam 54. The signal beam 54 thus passes through the oscillator cavity without reflection, while the idler beam 52 is resonated to assure maximum output at the mid-IR wavelengths. Preferably, mirror 16 is also coated for high reflectance at the pump wavelengths between 1.0 and 1.1 µm. It is not, however, essential to provide this last high reflectance but such reflection may be advantageous for more efficient operation of the device by lowering the energy threshold for the parametric process.

An alternative configuration to the SRO is that of a Doubly Resonant Oscillation (DRO), where both the idler and signal waves are resonated. In general, a DRO is known to have a lower oscillation threshold but has the drawback of more complicated mirror coatings, and somewhat more difficult alignment procedures. Nonetheless, while an SRO is preferred due to greater simplicity and lower cost of components, DRO configurations are considered an alternative embodiment for cases where a substantially reduced oscillation threshold presents an advantage. It should be noted that while DRO outputs are known to be less stable than those of an SRO, this is not an issue for this present application where only pump beams comprising a multiplicity of longitudinal modes are utilized. A DRO is therefore an acceptable variation in all the OPO configurations discussed herein.

The surfaces of mirrors 12 and 16 may be flat, concave or convex, as would be apparent to a person of ordinary skill. In the preferred embodiment, flat surfaces are advantageous for converting multimode pump radiation, because mode matching would then be dominated by the pump beam 50, rather than the OPO cavity. Efficiency reduction due to higher order transverse modes is not as severe in this case. Since the resonator mode of a plane parallel OPO consists of a beam of parallel light, a lens to focus the pump beam is also not required, thereby resulting in further simplification of the overall OPO laser design. Alternatives using concave-convex surfaces are possible, but are somewhat more complex to align, as a lens would then have to be provided to match the waist of the pump to the small waist of the OPO resonator mode, further requiring a single transverse-mode pump to assure high OPO efficiency. Mode matching is an important consideration in this type of configuration since any mode mismatch will cause a reduction in gain for optical parametric oscillation and a subsequent increase in threshold. In the preferred embodiment, a less complex and cheaper pump laser would provide a multi-mode beam, with the limits on allowed divergence dictated by the needs of the delivery system rather than the OPO.

The pump laser 20 consists generally of a neodymium-doped laser rod, such as Nd:YAG, pumped by either flashlamps or diode arrays. Both flashlamp and diode pumped lasers of the required energy, peak power and repetition rate are well known and commercially available. Other appropriate laser media include crystals such as Nd:YLF, Nd:glass and Nd:YAlO₃, all of which provide the fundamental radiation at wavelengths falling in the range covered by the present application.

The crystal 15 preferably comprises a nonlinear material having high nonlinear coefficient, reasonably wide angular and temperature bandwidths, high damage threshold and minimal absorption at the idler or signal wavelengths. Ideally, a crystal that can be phase-matched non-critically would be preferred, since that would result in the largest possible walk-off angles allowing laser beams with even poor beam quality to be readily converted in long crystals. In a non-critical phase matching (NCPM) arrangement, the crystal is oriented such that phase matching is achieved along a propagation direction parallel to one of the crystal's principal axes (X, Y, or Z). In practice, it may not be possible with currently available materials and lasers to fulfill this criteria for a given application. Alternatively, a crystal with critical phase matching (CPM) may be acceptable as long as the walk-off angles and angular bandwidths are sufficiently high to allow efficient conversion of beams that are not necessarily single transverse mode. We have determined that the crystal known as Potassium Titanyl Phosphate (KTiOPO₄ or "KTP") is capable of fulfilling the requirements of this application, even though KTP could not be non-critically phase matched with the idler wavelengths of choice generated under pumping with a 1.06 µm laser. The KTP crystal is also known to exhibit some absorption at or near 3 microns, usually attributed to the presence of residual OH- radicals inherent to the growth process. Such absorption, if overly large, would seem to hinder the use of KTP for higher repetition rate applications.

We have determined, however, that under the right conditions, KTP is suitable asan OPO crystal for the corneal sculpting application, even with the level of absorption present with current material growth capability. As discussed below, this has been achieved by the fortuitous combination of KTP's large temperature bandwidth and modest energy output and average power requirements of the surgical applications contemplated. With a crystal cut for Type-II phase matching, internal angles of 68 to 70 degrees would provide the required wavelengths for the idler when pumped by a 1.064 µm Nd:YAG laser, based on known material parameters for x-cut material. These angles may be sufficiently close enough to 90° to provide acceptance angles large enough to accommodate multi-mode pump beams with divergence exceeding many times the diffraction limit, if required. It is to be understood, however, that a judicious selection of components is necessary to achieve the operational conditions required of the surgical laser instrument, especially when the criterion of a compact, simple device consistent with portability in the field is factored in. Measured against the stringent parameters imposed by, for example, the corneal sculpting application, the particular combinations of various OPO elements and parameters using available materials and optics in the simple optical arrangement depicted in Figure 1 was not apriori obvious.

Accordingly, in one key aspect of this invention, a KTP crystal of sufficient length must be selected to allow efficient conversion of the 1 micron radiation. In a preferred embodiment, crystal lengths of at least 20 mm but potentially as long as 30 mm are appropriate, based on trade-offs of the walk-off angles that are realizable in a 68 to 70° Type-II CPM configuration for the x-cut crystal and estimates of the OPO gain required to produce idler output energy levels in the desired 5 to 30 mJ range. At this orientation, the acceptance angle for KTP is on the order of 5 cm-mrad, which is still large enough to accommodate the multi-mode pump preferred for the present application.

It is also to be understood that the specific wavelength of the output beam 52 can be altered by rotating the crystal with respect to the principal axes. This is a potentially useful feature in the surgical context since absorption properties may differ among different types of tissues and, for example, even within the same tissue, as a function of temperature. Hence, a slight variation of wavelength could allow matching to the optimal absorption desired for a given procedure, thus enlarging the scope and utility of the OPO laser source. The limitation on the wavelength range that can be so obtained is determined by the relative sizes of the pump beam and the crystal aperture. Based on known parameters of KTP and the crystal sizes that are readily available, a wavelength range extending from 2.75 to just over 3 µm can all be covered with the present configuration, using any one of several commercially available neodymium-doped pump lasers.

Yet another important aspect of the invention relates to utilization of sufficiently short pump laser pulses such that OPO thresholds may be reached even with an unfocused pump beam arrangement. By eliminating the need for focusing the beam into the crystal, multimode or unstable resonator pump beam spatial distributions may be utilized, which has the advantage of significantly relaxing the requirements for a pump laser while alleviating difficulties associated with the OPO mode matching. In the preferred embodiment, pump pulse durations (FWHM) between 5 ns and 12 ns were found to be acceptable, producing efficient conversion to the idler's wavelengths of over 10% even for a multimode pump beam with divergence greater than 8 times the diffraction limit.

In another feature of the invention, bare crystal faces (i.e., non-antireflection (AR) coated) could be used to alleviate risk of damage associated with deficiencies of current coating technologies, whereby residual absorption near the 3 micron wavelength of choice can lower damage thresholds to impractical levels especially when short-duration pulses are utilized. Should high quality, 3 micron coatings become available for KTP, they could be used to advantage as this would lower the OPO losses and allow further reduction in the threshold for parametric oscillation for the same slope efficiency. It should be pointed out, however, that for optimal performance and damage-free operation, the threshold should be such that the desired idler energy output is achieved with an input energy of no more than 3-4 times the threshold. By AR-coating the crystal, the reflectivity of the output coupler can be decreased, thereby dropping the circulating 2.9 µm power for the same output energy.

In the example quoted above, it was determined that with a bare crystal, damage to either the crystal or the optics could be avoided even with input pump energies in excess of 250 mJ for a 10 Hz beam, using all standard optics. Again, the ability to use unfocused beams with diameters on the order of 1 to 5 mm is considered a critical aspect in achieving this performance. To further suppress the potential for damage, especially on the input mirror which is subjected to the full pump power, other arrangements can be employed whereby the pump beam is not coupled through the same 0° input mirror that must also provide high reflection at 3 microns. There are indications that reflecting the 3 micron idler beam at 45° instead can increase the damage threshold when the best available 1 micron coatings are used.

Referring now to Figure 2, an alternative embodiment is illustrated, in which an "L-shaped" cavity is employed using the three mirrors indicated as 16, 17 and 18 to provide some separation between the path of the pump beam 50 and the idler beam 52. Thus, the pump is coupled through a 45° mirror 17 which is coated to also provide high reflection (at 45°) at the idler wavelengths. Mirror 18 is also coated to reflect the idler beam 52, but it is not subjected to the high power pump beam 50. The idler beam 52 is then coupled out through mirror 16, which is partially reflecting at the wavelength of the idler beam 52. Again, as in Figure 1, mirror 16 is preferably coated to provide back reflection of the pump beam 50, to lower the threshold for the parametric process. The advantage of this "L" cavity is that the fluence on the input mirror is reduced due to the 45° angle of incidence. Since this mirror 17 is typically the first component to damage, lower fluence translates into reduced probability of damage to the OPO at a given level of energy output.

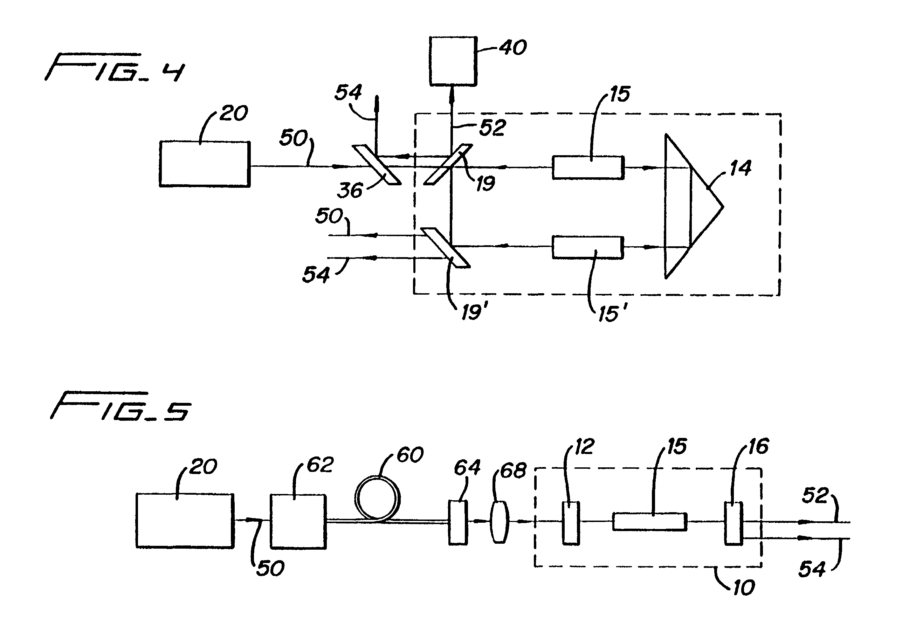

It is to be noted that in the embodiments of both Figures 1 and 2, the OPO axis must be slightly offset from the pump axis to prevent feedback into the pump laser 20. As an alternative, an isolator can be used between the pump laser and the OPO, although that would result in additional cost to the system. Figures 3 and 4 represent two alternative configurations that have no pump feedback as they rely on single-pass pumping. Thus, to increase conversion and reduce threshold, instead of back reflection of the pump into the same crystal, two OPO crystals are used in tandem. Figure 3 shows an arrangement whereby the pump beam 50 is coupled into the OPO cavity through a 45° mirror 11 that is coated for high reflection at the pump wavelengths and high transmission at the idler wavelengths. The pump beam passes through two nonlinear crystals 15' and 15" and is then transmitted out of the cavity through a mirror 12 that is coated for high transmission at the pump wavelength and high reflection at the 3.0 µm wavelength range of the idler beam 52. The idler beam 52 is coupled out of the cavity through a mirror 13 that is coated to partially reflect the idler wavelengths with the reflectivity selected to optimize the output from the cavity. In this singly resonant oscillator (SRO), each of the mirrors 11, 12 and 13 are coated to transmit the signal wavelength so that only the idler wavelength is resonant. An alternative arrangement would utilize a DRO which requires reflective coatings at the signal wavelength as well, and possibly also an additional beam splitter and/or other optics. The threshold would then be lower, but at a cost of increased complexity to the optics and in alignment procedures.

Figure 4 depicts a so-called "ring" configuration, where a prism 14 provides total internal reflection (TIR) of the beams in the cavity to thus pump two OPO crystals, marked again as 15 and 15' in a single pass arrangement. Two 45° mirrors 19 and 19' are coated to provide high transmission at the pump and signal wavelengths. Mirror 19' is also coated to reflect the idler wavelength, while mirror 19 is partially reflective at 3 µm to outcouple the idler beam 52. As Figure 4 shows, the residual pump beam 50 is now exiting the OPO cavity via mirror 19', thus posing no feed-back problems. Also, since most of the signal beam 54 is transmitted out of the cavity also through mirror 19', there is less of a requirement for further attenuation of the signal in the path of the idler beam 52. While attractive on these last two counts, the configuration of Figure 4 is optically more complex, requiring additional elements as compared to the simple arrangement of Figure 1.

Figure 5 depicts substantially an alternative novel arrangement using a wave guide means 60 to couple the pump radiation into the OPO. In a preferred embodiment, the waveguide means comprises a hollow waveguide, a fiber or a fiber bundle. The advantages of using fiber delivery over an air path, fixed beam delivery system for a medical laser system are well known. They include easier alignment of the beam to the surgical site, more flexible adjustment of radiation, delivery angle and location, homogenization (or spatial smoothing) of a multimode beam and the ability to deliver radiation to internal locations not otherwise accessible. However, while fibers for transmitting 1 micron radiation are well developed with damage threshold that can withstand 100's of millijoules of short-pulse radiation, there are not similar fibers currently available to transmit short-pulse, 3 micron radiation. It would therefore be beneficial, if the higher power 1 micron pump beam could be transmitted over a fiber, allowing placement of the OPO in close proximity to the surgical microscope. Most of the advantages of a fiber delivery system would carry over when it is the pump light coupling through a fiber, with the exception of accessing internal locations. In particular, homogenization of the pump beam would result in a smoother profile for the output mid-IR beam, a highly desirable attribute in corneal ablation.

In the embodiment of Figure 5, the pump beam 50 is coupled through lenses 62 into a fiber 60, which may, in an alternative embodiment consist of a polarization preserving fiber bundle or a hollow metal waveguide. A bundle may be suitable for accepting and transmitting a divergent pump beam 50 efficiently while allowing for collection and recollimation of light at the distal end through standard optical means 64. A lens 68, is shown as imaging the pump light into the OPO. In a preferred embodiment, the lens provides 1:1 imaging, assuming a 6 mm diameter bundle, to preserve the characteristics of the unfocused pump beam arrangement. Other aspect ratios are feasible, depending on the characteristics of available pump beams and fiber numerical apertures. In the preferred embodiment, the bundle may consist of a number of polarization preserving single mode fibers, as required to allow phase matching in the OPO crystal. Using this method, the damage limit of each fiber and the divergence of the beam(s) exiting the fiber(s) must be addressed, as would be apparent to a person of ordinary skill. In the case of the hollow metal waveguide, there are indications that polarization may be preserved and that a waveguide with approximately I mm diameter can deliver well over 100 mJ short pulse light at 1 µm wavelength. Such optical means as needed to correct residual depolarization of the pump light exiting waveguide 60, may be included as part of optical element 64 in the schematic of Figure 5. For simplicity, only the simple OPO configuration of Figure 1 is illustrated in Figure 5, but it is to be understood that any of the alternative OPO embodiments of Figures 2 through 4 can be used as the OPO element 10 in Figure 5.

It is to be noted that absorption in the KTP crystal of choice at or near 3 microns can limit scaling the repetition frequency of the OPO laser source of any of the configurations depicted above. Thus, absorption levels of 8-10% through the length of the crystal were found to be acceptable for the below 0.5 W average power OPO outputs considered this far, a result attributed to the unusually wide temperature bandwidth of KTP. However, it is recognized that to scale the repetition rate of the OPO to beyond 40-50 Hz may require some progress in the material area, whereby growth can be done under altered conditions that do not favor formation of the absorbing OH⁻ ions. Such advances are currently contemplated, and should they be realized, would allow scaling the repetition rate to beyond the 50 Hz level. Additional scaling of the repetition frequency to the 100 Hz level can also be provided, for example, by interlacing the outputs of two OPOs, pumped by a single laser beam. These, as well as other arrangements utilizing a multiplicity of crystals, fall under the domain of the present invention.

Alternative KTP isomorphs such as KTA and RTA are also recognized as candidates for a mid-IR OPO laser using any one of the configurations specified above, given that they have similar properties to KTP. The selection of a particular crystal thus depends on a combination of characteristics, primarily related to favorable phase matching and minimal absorption at the wavelengths of choice for the present application.

Finally, there are a number of alternative OPO technologies that should they be developed in the near future could be used to advantage in the surgical OPO laser disclosed herein. Such improvements include use of a periodically-poled (PP) KTP which may provide drastically lower thresholds due to high nonlinearities. Output energies from a PP KTP are currently limited to less than 1 mJ due to small (<1 mm) apertures, but larger PP KTP crystals may become available through evolving technologies such as fusion bonding. Furthermore, in a periodically-poled form, LiNbO₃ pumped at 1 µm may also be a candidate crystal for producing the requisite 2.9-3.0 µm wavelengths under quasi phase-matching conditions which effectively simulate NCPM. Apertures are again limited to less than a mm, but future developments may result in larger PP crystals becoming available in the not too-distant future. Of course, absorption in LiNBO₃ at 3 micron remains a problem which will have to be addressed especially for higher repetitious rates.

We also note that utilization of a pump laser source with output wavelengths in the 0.85 to 0.9 µm range represents another alternative OPO configuration. With this pump wavelength, it is possible to non-critically phase-match KTP (x-cut), which would be highly beneficial to the surgical applications contemplated. Unfortunately, pump lasers providing such near-infrared radiation are not yet available as compact low cost, commercial lasers. Candidates include lamp-pumped Ti:sapphire and Cr:LiSAF, neither of which is readily available with the required energy (greater than 100 mJ), pulse duration (less than 25 ns), and repetition rate (greater than 10 Hz) capability. These or similar lasers may however be developed in the future and are thus included within the scope of this invention.

It is to be understood that the embodiments and variations shown and described herein are merely illustrative of the principles of this invention and that various modifications may be implemented by those skilled in the art without departing from the scope of the invention, which is defined by the claims.

## Claims

1. A mid-infrared laser system for performing a laser surgical procedure on a tissue, said system comprising:
a laser source means (20) for producing a pump beam (50) having a pulse duration of less than 50 ns and a wavelength ranging approximately from 0.85 to 0.9 µm or from 1.0 to 1.1 µm;
a non-linear crystal (15, 15', 15") for parametrically converting the pump beam (50) into an idler beam (52) and a signal beam (54), said idler beam (52) having a wavelength in the mid-infrared range corresponding approximately to an absorption peak of said tissue; and
means (40) for directing said idler beam (52) onto said tissue to remove portions of said tissue primarily by a photo-mechanical ablation process; and
wherein said system includes means comprising a beam dump (32) to prevent the signal wavelengths from the signal beam (54) from reaching said tissue.

2. The laser system according to claim 1, **characterized in that** said pump beam (50) has a pulse duration of less than 25 ns.

3. The laser system according to claim 1 or claim 2, **characterized in that** the system is adapted to generate a thermal damage zone that is limited to a sub-micron distance.

4. The laser system according one of claims 1 to 3, **characterized in that** said non-linear crystal (15, 15', 15") has a dimension of at least 20 mm.

5. The laser system according one of claims 1 to 4, **characterized in that** said non-linear crystal (15, 15', 15") is part of an optical parametric oscillator, the optical parametric oscillator having an axis offset from the pump axis sufficient to prevent feedback into said laser source means (20).

6. The laser system according to one of claims 1 to 5, **characterized in that** said idler beam (52) has a wavelength in the mid-infrared range approximately between 2.85 and 3.0 µm.

7. The laser system according to any of claims 1 to 6, **characterized in that** said laser source means (20) is a neodymium-doped laser.

8. The laser system according to any of claims 1 to 7, **characterized in that** said pump beam (50) has a repetition rate of at least 10 Hz and a transverse mode structure consisting of single or multiple modes.

9. The laser system according to any of claims 1 to 8, **characterized in that** said non-linear crystal (15, 15', 15") is a Potassium Titanyl Phosphate (KTP) crystal.

10. The laser system according to any of claims 1 to 9, **characterized in that** the non-linear crystal (15, 15', 15") is rotatable about three principal axes.

11. The laser system according to any of claims 1 to 10, **characterized in that** said non-linear crystal (15, 15', 15") is made of a periodically poled non-linear material including KTP and isomorphs or LiNbO₃.

12. The laser system according to any of claims 1 to 11, **characterized in that** said non-linear crystal (15, 15', 15") is tunable to optimize absorption in said tissue.

13. The laser system according to any of claims 1 to 12, **characterized in that** said idler beam (52) has energy output of at least 1 mJ.

14. The laser system according to any of claims 1 to 13, **characterized in that** said idler beam (52) achieves a thermal damage zone in corneal tissue of less than 2 µm.

15. The laser system according to any of claims 1 to 14, **characterized in that** said directing means includes three mirrors (16, 17, 35) comprising an "L shaped" arrangement.

16. The laser system according to any of claims 1 to 15, **characterized in that** the non-linear crystal is based on a doubly-resonant oscillator.

17. The laser system according to any of claims 1 to 16, **characterized by** a pair of said non-linear crystals (15, 15') pumped by said laser source means (20) with interlaced beams whereby an overall repetition rate of at least 20 Hz is achieved.

18. The laser system according to any of claims 1 to 17, **characterized in that** the fluence onto the tissue is between 100 mJ/cm² and 500 mJ/cm².

19. The laser system according to any of claims 1 to 18, **characterized in that**
said non-linear crystal (15, 15', 15") is rotatable about three principal axes for parametrically converting the pump beam (50) into said idler beam (52) and said signal beam (54);
said idler beam (52) has a wavelength in the mid-infrared range approximately between 2.85 and 3.0 µm; and
said non-linear crystal (15, 15', 15") is non-critically phase matched and said crystal (15, 15', 15") is oriented such that phase matching is achieved along a propagation direction of said idler beam (52) parallel to one of said principal axes.

20. The laser system according to any of claims 1 to 19, **characterized in that** said laser source means (20) produces a pump beam (50) having a defined polarization; fiber means (60) is provided for coupling said laser source (20) to said nonlinear crystal (15), said fiber means (60) maintaining said polarization.

21. The laser system according to any of claims 1 to 20, wherein said tissue is a tissue of the eye.

22. The laser system according to claim 21, wherein said tissue of the eye is corneal tissue.

23. The laser system according to any of claims 1 to 22 adapted for use for removing corneal tissue from an eye of a patient by a PRK technique based on a photospallation mechanism.

24. The laser system of any one of claims 1 - 23, **characterized in that** said means to prevent the signal wavelengths from the signal beam (54) from reading said tissue comprise an attenuator (34) placed in the path of the idle beam (52).

## Patentansprüche

1. Ein Mittel-Infrarot-Laser-System zum Ausführen eines Laser-Operations-Verfahrens an einem Gewebe, wobei das System umfasst:
eine Laser-Quellen-Vorrichtung (20) zum Erzeugen eines Pump-Strahls (50), welcher eine Puls-Dauer von weniger als 50 ns und eine Wellenlänge ungefähr im Bereich von 0,85 bis 0,9 µm oder von 1,0 bis 1,1 µm aufweist;
einen nichtlinearen Kristall (15, 15', 15") zum parametrischen Konvertieren des Pump-Strahls (50) in einen Idler-Strahl (52) und einen Signal-Strahl (54), wobei der Idler-Strahl (52) eine Wellenlänge im mittleren Infrarot-Bereich aufweist, welche ungefähr einer Absorptions-Spitze des Gewebes entspricht; und
einer Vorrichtung (40) zum Leiten des Idler-Strahls (52) auf das Gewebe zum Entfernen von Teilen dieses Gewebes im Wesentlichen mittels eines photomechanischen Ablations-Prozesses; und
wobei das System Mittel enthält, welche einen Strahl-Auffänger (32) umfassen, um die Signal-Wellenlängen des Signal-Strahls (54) daran zu hindern, das Gewebe zu erreichen.

2. Das Laser-System gemäß Anspruch 1, **dadurch gekennzeichnet, dass** der Pump-Strahl (50) eine Puls-Dauer von weniger als 25 ns aufweist.

3. Das Laser-System gemäß Anspruch 1 oder Anspruch 2, **dadurch gekennzeichnet, dass** das System dazu eingerichtet ist, eine thermische Schadenszone zu erzeugen, welche auf einen Sub-Mikrometer-Abstand begrenzt ist.

4. Das Laser-System gemäß einem der Ansprüche 1 bis 3, **dadurch gekennzeichnet, dass** der nichtlineare Kristall (15, 15', 15") ein Ausmaß von wenigstens 20 mm aufweist.

5. Das Laser-System gemäß einem der Ansprüche 1 bis 4, **dadurch gekennzeichnet, dass** der nichtlineare Kristall (15, 15', 15") Teil eines optischen parametrischen Oszillators ist, wobei der optische parametrische Oszillator eine Achse aufweist, die gegenüber der Pump-Achse ausreichend versetzt ist, um eine Rückkopplung in die Laser-Quellen-Vorrichtung (20) zu verhindern.

6. Das Laser-System gemäß einem der Ansprüche 1 bis 5, **dadurch gekennzeichnet, dass** der Idler-Strahl (52) eine Wellenlänge im Mittel-Infrarot-Bereich ungefähr zwischen 2,85 und 3,0 µm aufweist.

7. Das Laser-System gemäß einem der Ansprüche 1 bis 6, **dadurch gekennzeichnet, dass** die Laser-Quellen-Vorrichtung (20) ein Neodym-dotierter Laser ist.

8. Das Laser-System gemäß einem der Ansprüche 1 bis 7, **dadurch gekennzeichnet, dass** der Pump-Strahl (50) eine Repetitions-Rate von wenigstens 10 Hz und eine transversale Moden-Struktur aufweist, welche aus Einzel-Moden oder Mehrfach-Moden besteht.

9. Das Laser-System gemäß einem der Ansprüche 1 bis 8, **dadurch gekennzeichnet, dass** der nichtlineare Kristall (15, 15', 15") ein Kalium-Titanyl-Phosphat (KTP) Kristall ist.

10. Das Laser-System gemäß einem der Ansprüche 1 bis 9, **dadurch gekennzeichnet, dass** der nichtlineare Kristall (15, 15', 15") um drei Hauptachsen drehbar ist.

11. Das Laser-System gemäß einem der Ansprüche 1 bis 10, **dadurch gekennzeichnet, dass** der nichtlineare Kristall (15, 15', 15") aus einem periodisch gepolten nichtlinearen Material hergestellt ist, welches KTP und Isomorphe oder LiNbO₃ enthält.

12. Das Laser-System gemäß einem der Ansprüche 1 bis 11, **dadurch gekennzeichnet, dass** der nichtlineare Kristall (15, 15', 15") abstimmbar ist, um die Absorption in dem Gewebe zu optimieren.

13. Das Laser-System gemäß einem der Ansprüche 1 bis 12, **dadurch gekennzeichnet, dass** der Idler-Strahl (52) eine Energie-Ausgabe von wenigstens 1 mJ aufweist.

14. Das Laser-System gemäß einem der Ansprüche 1 bis 13, **dadurch gekennzeichnet, dass** der Idler-Strahl (52) in Hornhaut-Gewebe eine thermische Schadenszone von weniger als 2 µm aufweist.

15. Das Laser-System gemäß einem der Ansprüche 1 bis 14, **dadurch gekennzeichnet, dass** die Leit-Vorrichtung drei Spiegel (16, 17, 35) enthält, welche eine "L-förmige" Anordnung umfassen.

16. Das Laser-System gemäß einem der Ansprüche 1 bis 15, **dadurch gekennzeichnet, dass** der nichtlineare Kristall auf einem doppelt resonanten Oszillator basiert.

17. Das Laser-System gemäß einem der Ansprüche 1 bis 16, **gekennzeichnet durch** ein Paar der nichtlinearen Kristalle (15, 15'), welche von der Laser-Quellen-Vorrichtung (20) mit verschachtelten Strahlen gepumpt werden, wobei eine Gesamt-Repetitions-Rate von wenigstens 20 Hz erreicht wird.

18. Das Laser-System gemäß einem der Ansprüche 1 bis 17, **dadurch gekennzeichnet, dass** der Fluss auf das Gewebe zwischen 100 mJ/cm² und 500 mJ/cm² beträgt.

19. Das Laser-System gemäß einem der Ansprüche 1 bis 18, **dadurch gekennzeichnet, dass**
der nichtlineare Kristall (15, 15', 15") um drei Hauptachsen drehbar ist, um den Pump-Strahl (50) parametrisch in den Idler-Strahl (52) und den Signal-Strahl (54) zu konvertieren;
der Idler-Strahl (52) eine Wellenlänge im Mittel-Infrarot-Bereich von ungefähr zwischen 2,85 bis 3,0 µm aufweist; und
der nichtlineare Kristall (15, 15', 15") in nicht-kritischer Weise phasen-angepasst ist, und dass dieser Kristall (15, 15', 15") derart orientiert ist, dass Phasen-Anpassung entlang einer Propagations-Richtung des Idler-Strahls (52) erreicht wird, welche zu einer der Hauptachsen parallel ist.

20. Das Laser-System gemäß einem der Ansprüche 1 bis 19, **dadurch gekennzeichnet, dass** die Laser-Quellen-Vorrichtung (20) einen Pump-Strahl (50) erzeugt, welcher eine definierte Polarisation aufweist; dass eine Faser-Vorrichtung (60) bereitgestellt ist, um die Laser-Quelle (20) an den nichtlinearen Kristall (15) zu koppeln, wobei die Faser-Vorrichtung (60) die Polarisation erhält.

21. Das Laser-System gemäß einem der Ansprüche 1 bis 20, wobei das Gewebe ein Gewebe des Auges ist.

22. Das Laser-System gemäß Anspruch 21, wobei das Gewebe des Auges Hornhaut-Gewebe ist.

23. Das Laser-System gemäß einem der Ansprüche 1 bis 22, welches zur Anwendung zur Entfernung von Hornhaut-Gewebe von einem Auge eines Patienten mittels einer auf einem Photo-Spallations-Mechanismus basierenden PRK-Technik eingerichtet ist.

24. Das Laser-System gemäß einem der Ansprüche 1 bis 23, **dadurch gekennzeichnet, dass** die Vorrichtung zum Verhindern, dass die Signal-Wellenlängen des Signal-Strahls (54) das Gewebe erreichen, einen im Pfad des Idler-Strahls (52) angeordneten Abschwächer (34) umfassen.

## Revendications

1. Système laser dans l'infrarouge moyen pour exécuter une intervention chirurgicale au laser sur un tissu, ledit système comprenant :
des moyens de source laser (20) pour produire un faisceau de pompage (50) ayant une durée d'impulsions de moins de 50 ns et une longueur d'onde s'étageant approximativement de 0,85 à 0,9 µm, ou de 1,0 à 1,1 µm ;
un cristal non linéaire (15, 15', 15") pour convertir de façon paramétrique le faisceau de pompage (50) en un faisceau de renvoi (52) et un faisceau de signal (54), ledit faisceau de renvoi (52) ayant une longueur d'onde dans la plage de l'infrarouge moyen correspondant approximativement à un pic d'absorption dudit tissu ; et
des moyens (40) pour diriger ledit faisceau de renvoi (52) sur ledit tissu afin de supprimer des parties dudit tissu principalement au moyen d'un processus d'ablation photomécanique ; et
dans lequel ledit système comprend des moyens comprenant une décharge de faisceau (32) pour empêcher que les longueurs d'ondes de signal en provenance du faisceau de signal (54) ne parviennent jusqu'au dit tissu.

2. Système laser selon la revendication 1, **caractérisé en ce que** ledit faisceau de pompage (50) a une durée d'impulsions de moins de 25 ns.

3. Système laser selon la revendication 1 ou 2, **caractérisé en ce que** le système est adapté pour générer une zone de dommage thermique qui est limitée à une distance inférieure au micron.

4. Système laser selon l'une des revendications 1 à 3, **caractérisé en ce que** ledit cristal non linéaire (15, 15', 15") a une dimension d'au moins 20 mm.

5. Système laser selon l'une des revendications 1 à 4, **caractérisé en ce que** ledit cristal non linéaire (15, 15', 15") fait partie d'un oscillateur paramétrique optique, l'oscillateur paramétrique optique ayant un décalage de son axe par rapport à l'axe de la pompe qui est suffisant pour empêcher toute rétroaction à l'intérieur desdits moyens de source laser (20).

6. Système laser selon l'une des revendications 1 à 5, **caractérisé en ce que** ledit faisceau de renvoi (52) a une longueur d'onde dans la plage de l'infrarouge moyen qui est comprise entre 2,85 et 3,0 µm approximativement.

7. Système laser selon l'une quelconque des revendications 1 à 6, **caractérisé en ce que** lesdits moyens de source laser (20) sont un laser dopé au néodyme.

8. Système laser selon l'une quelconque des revendications 1 à 7, **caractérisé en ce que** ledit faisceau de pompage (50) a une cadence de répétition d'au moins 10 Hz et une structure en mode transversal composée d'un seul ou de plusieurs modes.

9. Système laser selon l'une quelconque des revendications 1 à 8, **caractérisé en ce que** ledit cristal non linéaire (15, 15', 15") est un cristal de potassium titanyl phosphate (KTP).

10. Système laser selon l'une quelconque des revendications 1 à 9, **caractérisé en ce que** le cristal non linéaire (15, 15', 15") est susceptible de tourner autour de trois axes principaux.

11. Système laser selon l'une quelconque des revendications 1 à 10, **caractérisé en ce que** ledit cristal non linéaire (15, 15', 15") est fait en un matériau non linéaire périodiquement polarisé, comprenant du KTP et des isomorphes ou du LiNbO₃.

12. Système laser selon l'une quelconque des revendications 1 à 11, **caractérisé en ce que** ledit cristal non linéaire (15, 15', 15") est accordable de façon à optimiser la capacité d'absorption dudit tissu.

13. Système laser selon l'une quelconque des revendications 1 à 12, **caractérisé en ce que** ledit faisceau de renvoi (52) délivre en sortie une énergie d'au moins 1 mJ.

14. Système laser selon l'une quelconque des revendications 1 à 13, **caractérisé en ce que** ledit faisceau de renvoi (52) réalise une zone de dommage thermique de moins de 2 µm dans le tissu cornéen.

15. Système laser selon l'une quelconque des revendications 1 à 14, **caractérisé en ce que** lesdits moyens de direction comprennent trois miroirs (16, 17, 35) disposés selon un agencement "en forme de L".

16. Système laser selon l'une quelconque des revendications 1 à 15, **caractérisé en ce que** le cristal non linéaire est basé sur un oscillateur doublement résonant.

17. Système laser selon l'une quelconque des revendications 1 à 16, **caractérisé par** une paire desdits cristaux non linéaires (15, 15') qui est pompée par lesdits moyens de source laser (20) avec des faisceaux entrelacés, moyennant quoi une cadence de répétition globale d'au moins 20 Hz est obtenue.

18. Système laser selon l'une quelconque des revendications 1 à 17, **caractérisé en ce que** la fluence sur le tissu se situe entre 100 mJ/cm² et 500 mJ/cm².

19. Système laser selon l'une quelconque des revendications 1 à 18, **caractérisé en ce que** :
ledit cristal non linéaire (15, 15', 15") est susceptible de tourner autour de trois axes principaux de façon à convertir de façon paramétrique le faisceau de pompage (50) en ledit faisceau de renvoi (52) et ledit faisceau de signal (54) ;
ledit faisceau de renvoi (52) a une longueur d'onde dans la plage de l'infrarouge moyen comprise entre 2,85 et 3,0 µm approximativement ; et
ledit cristal non linéaire (15, 15', 15") est en synchronisation de phase de façon non critique et ledit cristal (15, 15', 15") est orienté de telle sorte que la synchronisation de phase soit obtenue le long d'une direction de propagation dudit faisceau de renvoi (52) parallèlement à un desdits axes principaux.

20. Système laser selon l'une quelconque des revendications 1 à 19, **caractérisé en ce que** lesdits moyens de source laser (20) produisent un faisceau de pompage (50) ayant une polarisation définie ;
des moyens de fibres (60) sont fournis pour raccorder ladite source laser (20) audit cristal non linéaire (15), lesdits moyens de fibres (60) maintenant ladite polarisation.

21. Système laser selon l'une quelconque des revendications 1 à 20, dans lequel ledit tissu est un tissu de l'oeil.

22. Système laser selon la revendication 21, dans lequel ledit tissu de l'oeil est du tissu cornéen.

23. Système laser selon l'une quelconque des revendications 1 à 22 adapté pour éliminer du tissu cornéen de l'oeil d'un patient suivant une technique PRK basée sur un mécanisme de photospallation.

24. Système laser selon l'une quelconque des revendications 1 à 23, **caractérisé en ce que** lesdits moyens pour empêcher que les longueurs d'ondes de signal en provenance du faisceau de signal (54) ne parviennent jusqu'au tissu comprennent un affaiblisseur (34) placé sur le trajet du faisceau de renvoi (52).
